# EUROPÄISCHE PATENTSCHRIFT

(11) **EP 1 548 029 B1**
(45) Veröffentlichungstag und Bekanntmachung des Hinweises auf die Patenterteilung: **15.04.2009**
(21) Anmeldenummer: 04011492.8
(22) Anmeldetag: 14.05.2004
(51) Int. Cl.: C07K 7/64, A61K 38/12, A61L 27/22

(54) **Peptid- und Peptidmimetika-Derivate mit Integrin-Inhibitor-Eigenschaften III**
Peptid- and peptide mimetic derivatives having integrin inhibitor properties III
Peptides et dérivés de peptides mimétiques possedant des propriétés d'inhibiteur d'integrine III

(30) Priorität: 02.06.2003 DE 10325049
(43) Veröffentlichungstag der Anmeldung: 29.06.2005
(73) Patentinhaber: Biomet Deutschland GmbH, 14167 Berlin (DE)
(72) Erfinder: Enderle, Anja, Dr., 64376 Mühltal (DE); Nies, Berthold, Dr., 64407 Fränkisch-Crumbach (DE); Kessler, Horst, Prof. Dr., 85748 Garching (DE); Auerheimer, Jörg, 81379 München (DE); Hersel, Ulrich, 69121 Heidelberg (DE); Dahmen, Claudia, 85356 Freising (DE)
(74) Vertreter: Baumgärtel, Gunnar

(56) Entgegenhaltungen:
- DE-A1- 10 040 105
- NAKHLE B M ET AL: "Synthesis of 3,5-Bis(phosphonomethyl)benzoic Acid and Its Application as a Metal Oxide Surface Bivalent Anchor" TETRAHEDRON, ELSEVIER SCIENCE PUBLISHERS, AMSTERDAM, NL, Bd. 55, Nr. 10, 5. März 1999 (1999-03-05), Seiten 2835-2846, XP004157121 ISSN: 0040-4020
- TRAMMELL ET AL.: INORGANIC CHEMISTRY, Bd. 38, Nr. 16, 1999, Seiten 3665-3669, XP001206811
- Auernheimer et al. (2005) ChemBioChem 6, 2034-2040.
- Auernheimer & Kessler (2006) Bioorg. Med. Chem. Lett. 16, 271-273.

## Beschreibung

Die Erfindung betrifft ein Verfahren zur Herstellung von Verbindungen der Formel 1

B-Q-X1 I

worin
- B: ein bioaktives, zelladhäsionsvermittelndes Molekül
- Q: fehlt oder ein organisches Spacer-Molekül und
- X₁: ein Ankermolekül, ausgewählt aus der Gruppe

-W (i)

-V-W (ii)

-V-[V-W₂]₂ (iii)

oder

-V-[V-(V-W₂)₂]₂ (iv),

wobei W 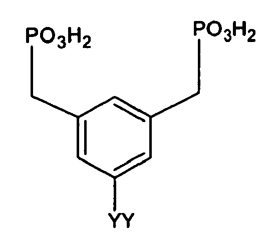 und V Lys, Asp oder Glu
   bedeutet, YY ist eine Carboxylgruppe. Eine freie Aminogruppe der Gruppe B wird mit einer freien Carboxylgruppe des
   Spacer-Moleküls Q oder des Ankermoleküls X₁, bzw. eine freie Aminogruppe des Restes Q mit einer freien Carboxylgruppe des Restes X₁ peptidartig verknüpft,
   sowie deren Salze.
   Ähnliche Verbindungen sind aus DE 10040105, DE 19932796, DE 19755800 und DE 19831710 bekannt.

Der Erfindung lag die Aufgabe zugrunde, ein Verfahren zur Herstellung der neuen Verbindungen mit wertvollen Eigenschaften bereitzustellen, insbesondere solche, die zur Herstellung von Arzneimitteln verwendet werden können, wobei das Verfahren eine kostengünstige und schnelle Synthese der Verbindungen ermöglicht.

Es wurde gefunden, dass die Verbindungen der Formel kostengünstig und schnell herstellbar sind, indem man das bioaktive Molekül B, welches mit Schutzgruppen versehen sein kann, und das mit Schutzgruppen und Phosphonestergruppen versehene Spacer-Anker-Molekül (Q-X₁) bzw. Anker-Molekül (X₁) peptidisch miteinander verknüpft und anschließend die Schutzgruppen gleichzeitig mit den Phosphonestergruppen abspaltet, und/oder dass man eine basische oder saure Verbindung der Formel durch Behandeln mit einer Säure oder Base in eines ihrer Salze überführt, wobei die Phosphonestergruppen 5-Carboxy-m-xylen-bisphosphonsäure-tetrabenzylester-Gruppen sind.

Es wurde ebenfalls gefunden, daß die Verbindungen der Formel 1 und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der αᵥβ₃- oder αᵥβ₅-Integrin-Rezeptoren mit Liganden hemmen, wie z. B. die Bindung von Fibrinogen an den β₃-Integrinrezeptor. Besondere Wirksamkeit zeigen die Verbindungen im Fall der Integrine αᵥβ₃, αᵥβ₅. α_{IIb}β₃ sowie αᵥβ₁ αᵥβ₆ und αᵥβ₈.
Diese Wirkung kann z.B. nach der Methode nachgewiesen werden, die von J.W. Smith et al. in J. Biol. Chem. 265, 12267-12271 (1990) beschrieben wird.

Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Verbindungen der Formel 1, die die Wechselwirkung von Integrinrezeptoren und Liganden, wie z. B. von Fibrinogen an den Fibrinogenrezeptor (Glycoprotein Ilb/Illa) blockieren, verhindern als GPllb/llla-Antagonisten die Ausbreitung von Tumorzellen durch Metastase. Dies wird durch folgende Beobachtungen belegt:
Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Der Phosphonatrest dient dazu, die Peptide ionisch oder adsorptiv an biokompatible Oberflächen von z.B. Implantaten zu binden, die Oxide aufweisen, wie z.B. Metalloberflächen (z.B. Titan bzw. Titanlegierungen wie TiAl₆V₄) bzw. Kationen-haltige Oberflächen wie z.B. auf amorphe oder gesinterte Calciumphosphate (z.B. Hydroxylapatit, Knochen, Zähne) oder Calciumphosphatzemente (z.B. Biocement D).

Gegenstand der Erfindung ist daher auch ein Verfahren zur Herstellung insbesondere die Verbindungen der Formel 1 zur ionischen oder adsorptiven Bindung über die funktionelle Gruppe des Restes X₁ an biokompatible Oberflächen.

Die erfindungsgemäßen Peptide ermöglichen nun die Biofunktionalisierung von Biomaterialien, insbesondere Implantaten für humane und tierische Organe durch deren Beschichtung, wobei vorwiegend die Adhäsion derjenigen Zellspezies stimuliert wird, die jeweils die Gewebeintegration des entsprechenden Biomaterials vollführen sollen. Mit der Verwendung solcher Beschichtungen ist eine beschleunigte und die verstärkte Integration verschiedener Biomaterialien/Implantate mit verbesserter Langzeitstabilität nach deren Einbringen in den Körper zu erzielen.

Die erfindungsgemäßen Peptide binden selektiv an Integrine. Nach Immobilisierung an biokompatiblen Oberflächen, z.B. Implantaten, stimulieren sie die Adhäsion von Zellen, die Integrine tragen. Nach Beschichtung der Verbindungen auf den Oberflächen, können selektiv diejenigen Zeit-Spezies zur Bindung stimuliert werden, die auch nach Implantation im natürlichen Gewebe die Implantatintegration vollführen sollen. So handelt es sich z.B. bei Osteoblasten, Osteoclasten und Endothelzellen um αᵥ-tragende Zeltspezies.

Gegenstand der Erfindung ist daher auch ein Verfahren zur Herstellung der Verbindungen der Formel 1 als Integrininhibitoren zur selektiven Zellanreicherung an Implantaten.

Die Verbindungen der Formel 1 können nach Verankerung an einer biokompatiblen Oberfläche als Arzneimittelwirkstoffe in der Human- und Veterinärmedizin eingesetzt werden, insbesondere können sie als Integrininhibitoren zur Behandlung von durch Implantate verursachten Erkrankungen, Defekten und Entzündungen wie ungenügender und verzögerter Integration von Biomaterialien und Implantaten, von durch Implantate verursachter Thrombose, von Knochen- und Zahndefekten, sowie von osteolytischen Erkrankungen wie Osteoporose, Thrombose, Herzinfarkt, Arteriosklerose, bei der Wundheilung zur Unterstützung der Heilungsprozesse, sowie zur Beschleunigung und Verstärkung des Integrationsprozesses des Implantats bzw. der biokompatiblen Oberfläche in das Gewebe, eingesetzt werden.

Die Verbindungen der Formel 1 können als antimikrobiell wirkende Substanzen bei Operationen eingesetzt werden, wo Biomaterialien, Implantate, Katheter oder Herzschrittmacher verwendet werden. Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

Entsprechende phosphonatankertragende Peptide können ionisch an Träger mit oxidhaltigen Oberflächen, wie z.B. Implantate, Affinitätschromatographien, oder Mikrotiterplatten oder auch an Kationen-haltige Oberflächen wie z.B. auf amorphe oder gesinterte Calciumphosphate (z. B. Hydroxylapatit, Knochen, Zähne) oder Calciumphosphatzemente (z.B. Biocement D) gebunden werden.

Gegenstand der Erfindung ist auch die Verwendung von Verbindungen der Formel 1 zur Beschichtung mittels ionischer oder adsorptiver Bindung von Implantaten für humane und tierische Organe.

Die vor- und nachstehend aufgeführten Abkürzungen von Aminosäureresten stehen für die Reste folgender Aminosäuren:
- Abu: 4-Aminobuttersäure
- Aha: 6-Aminohexansäure, 6-Aminocapronsäure
- Ala: Alanin
- Asn: Asparagin
- Asp: Asparaginsäure
- Arg: Arginin
- Cys: Cystein
- Dab: 2,4-Diaminobuttersäure
- Dap: 2,3-Diaminopropionsäure
- Gln: Glutamin
- Glp: Pyroglutaminsäure
- Glu: Glutaminsäure
- Gly: Glycin
- His: Histidin
- homo-Phe: homo-Phenylalanin
- Ile: Isoleucin
- Leu: Leucin
- Lys: Lysin
- Met: Methionin
- Nle: Norleucin
- Orn: Ornithin
- Phe: Phenylalanin
- Phg: Phenylglycin
- 4-Hal-Phe: 4-Halogen-phenylalanin
- Pro: Prolin
- Ser: Serin
- Thr: Threonin
- Trp: Tryptophan
- Tyr: Tyrosin
- Val: Valin.

Ferner bedeuten nachstehend:
- Ac: Acetyl
- BOC: tert.-Butoxycarbonyl
- CBZ oder Z: Benzyloxycarbonyl
- DCCI: Dicyclohexylcarbodiimid
- DMF: Dimethylformamid
- EDCI: N-Ethyl-N,N'-(dimethylaminopropyl)-carbodiimid
- Et: Ethyl
- FCA: Fluoresceincarbonsäure
- FITC: Fluoresceinisothiocyanat
- Fmoc: 9-Fluorenylmethoxycarbonyl
- FTH: Fluoresceinthioharnstoff
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- HATU: O-(7-Azabenzotriazol-1-yl)-N,N,N',N'-Tetramethyluronium-hexafluorophosphat
- HONSu: N-Hydroxysuccinimid
- OtBu: tert.-Butylester
- Oct: Octanoyl
- OMe: Methylester
- OEt: Ethylester
- POA: Phenoxyacetyl
- Pbf: Pentamethylbenzofuranyl
- Pmc: 2,2,5,7,8-Pentamethylchroman-6-sulfonyl
- Sal: Salicyloyl
- Su: Succinyl
- TIPS: Triisopropylsilan
- TFA: Trifluoressigsäure
- TMSBr: Trimethylsilylbromid
- Trt: Trityl (Triphenylmethyl).

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Ferner können die Aminosäuren, z. B. als Bestandteil von Verbindungen der Formel I, mit entsprechenden an sich bekannten Schutzgruppen versehen sein. Vor allem Seitenkettenmodifikationen des Arginins, wie sie z.B. bei den nichtpeptidischen aᵥβ₃-Antagonisten vorgenommen wurden (z.B. durch R.Keenan et al., Abstr. Pap. 211th ACS National Meeting (New Orleans, USA) 1996, MEDI 236), können auch bei den Cyclopeptiden eingesetzt werden, wie z.B. Benzimidazolderivate anstelle der Guanidingruppe.

In die erfindungsgemäßen Verbindungen sind auch sogenannte Prodrug-Derivate eingeschlossen, d. h. mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Gegenstand der Erfindung ist ferner ein Implantat, geeignet für humane und tierische Organe, bestehend aus einer Trägermatrix und einer diese Matrix umhüllenden Schicht eines bioaktiven, zelladhäsionsvermittelnden Moleküls, wobei die umhüllende Schicht aus einer Verbindung der Formel I gebildet wird, und wobei zwischen Trägermatrix und dieser Verbindung eine ionische oder adsorptive Bindung vorliegt. Vorzugsweise besteht die Trägermatrix und/oder deren Oberfläche aus einem Metall bzw. Metalloxid. Besonders bevorzugt besteht die Trägermatrix und/ oder deren Oberfläche aus einem Knochen- oder Zahnersatzmaterial wie z.B. aus Calciumphosphat-Mischungen.

Gegenstand der Erfindung ist ferner ein Verfahren zur Herstellung von Verbindungen der Formel I nach Anspruch 1 sowie ihrer Salze, dadurch gekennzeichnet, daß man ein bioaktives Molekül B, welches mit Schutzgruppen versehen sein kann, und ein mit Schutzgruppen versehenes Spacer-Anker-Molekül (Q-X₁) bzw. Anker-Molekül (X₁) peptidisch miteinander verknüpft und anschließend die Schutzgruppen abspaltet, und/oder dass man eine basische oder saure Verbindung der Formel I durch Behandeln mit einer Säure oder Base in eines ihrer Salze überführt.

Vor- und nachstehend haben die Reste B, Q und X₁ die bei der Formel I angegebene Bedeutung, sofern nicht ausdrücklich etwas anderes angegeben ist.

B bedeutet vorzugsweise ein cyclo-(Arg-Gly-Asp-Z₁)-, 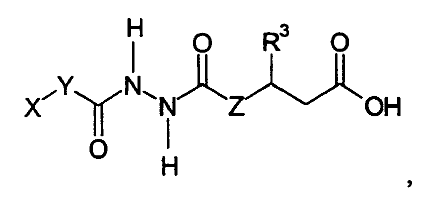 Thr-Trp-Tyr-Lys-Ile-Ala-Phe-Gln-Arg-Asn-Arg-Lys-,
Trp-Tyr-Lys-Ile-Ala-Phe-Gln-Arg-Asn-Arg-Lys-,
Tyr-Lys-Ile-Ala-Phe-Gln-Arg-Asn-Arg-Lys-,
Thr- Trp- Tyr-Lys-Ile-Ala-Phe-Gln-Arg-Asn-Arg-,
Thr-Trp-Tyr-Lys-Ile-Ala-Phe-Gln-Arg-Asn- oder ein
Thr-Trp-Tyr-Lys-Ile-Ala-Phe-Gln-Arg-Rest,
wobei Z₁ jeweils unabhängig voneinander einen Aminosäurerest oder einen Di- oder Tripeptidrest bedeutet, wobei die Aminosäuren unabhängig voneinander ausgewählt sind aus der Gruppe bestehend aus Ala, Asn, Asp, Arg, Cys, Gln, Glu, Gly, His, Homo-Phe, Ile, Leu, Lys, Orn, Met, Phe, Phg, Pro, Ser, Thr, Trp, Tyr, Val.

Q fehlt oder bedeutet ein organisches Spacer-Molekül. Bevorzugt ist dies ein [CO-(CH₂)ₓ-NH-]ₘ-, [CO-CH₂ (-O-CH₂CH₂)_{y}-NH-]ₘ-, [CO-(CH₂)_{z}-CO-]-, [NH-(CH₂)_{z}-NH-]-, [CO-CH₂-(OCH₂CH₂)_{y}-O-CH₂-CO-]- oder ein [NH-CH₂CH₂₋(OCH₂CH₂)_{y}-NH-]- Rest sowie deren Kombinationen, wobei für die Indices m, x, y und z die im Anspruch 3 beanspruchten Wertebereiche gelten. Als besonders vorteilhaft haben sich die vorgenannten Verbindungen erwiesen, die für m Werte zwischen 1 und 8, für x Werte zwischen 1 und 5 und für y und z Werte zwischen 1 und 6 annehmen können.

X₁ bedeutet ein Ankermolekül, vorzugsweise aus der Gruppe -W, -V-W, -V[V-W₂]₂ oder -V[V-(V-W₂)₂]₂.

Die in den Bedeutungen für Z₁ genannten Aminosäuren und Aminosäurereste können auch derivatisiert sein, wobei die N-Methyl-, N-Ethyl-, N-Propyl-, N-Benzyl- oder C_{α}-Methylderivate bevorzugt sind. Weiter bevorzugt sind Derivate von Asp und Glu, insbesondere die Methyl-, Ethyl, Propyl, Butyl, tert.-Butyl, Neopentyl- oder Benzylester der Seitenkettencarboxy-gruppen, ferner auch Derivate von Arg, das an der -NH-C(=NH)-NH₂-Gruppe mit einem Acetyl-, Benzoyl-, Methoxycarbonyl- oder Ethoxycarbonylrest substituiert sein kann.

X bedeutet vorzugsweise H₂N-C(=NH)-NH-, Het-NH-, H₂N-C(=NH)-, A-C(=NH)-NH oder ein Het-Rest.

Y bedeutet vorzugsweise 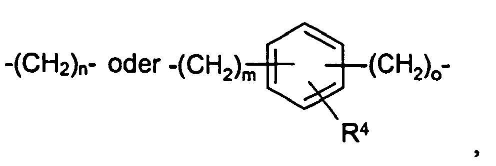 -(CH₂)ₛ-CH(R⁴)-(CH₂)ₜ- oder -(CH₂)ₚ-Het¹-(CH₂)_{q}-Rest.

Z bedeutet vorzugsweise N-R² oder CH-R², wobei R² vorzugsweise ein H-Atom oder Alkyl-Rest mit 1 bis 4 C-Atomen sein kann.

R³ bedeutet vorzugsweise ein H-Atom, Ar, Het oder A -Rest, wobei A, Ar und Het eine der zuvor oder nachstehend angegebenen Bedeutungen haben.

R⁴ bedeutet vorzugsweise ein H-Atom, A, Ar, OH, OA, OAr, Arylalkyl, Hal, CN, NO₂, CF₃ oder OCF₃ -Rest. Arylalkyl bedeutet bevorzugt Benzyl, Phenylethyl, Phenylpropyl oder Naphthylmethyl, besonders bevorzugt Benzyl.

A bedeutet vorzugsweise ein COOH-, NH₂ - oder Alkyl-Rest mit 1 bis 6 C-Atomen, unsubstituiert oder substituiert mit COOH oder NH₂. A bedeutet vorzugsweise Methyl, weiterhin Ethyl, Propyl, n-Butyl, Isobutyl, sek.-Butyl oder tert. Butyl, ferner auch n-Pentyl, 1-,2- oder 3-Methylbutyl, 1,1-, 1,2-oder 2,2-Dimethylpropyl, 1-Ethylpropyl, Hexyl, 1-,2-,3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Besonders bevorzugt für A ist Methyl.

Ar bedeutet vorzugsweise unsubstituiertes oder ein-, zwei- oder dreifach durch A, OH, OA, CF₃, OCF₃, CN, NO₂ oder Hal substituiertes Phenyl, welches durch ein-, zwei- oder dreifach durch A, OH, OA, NH₂, OCF₃, CN, NO₂ oder Hal substituiertes Phenyl in der Art substituiert sein kann, dass ein unsubstituiertes oder substituiertes Biphenyl entsteht.

Ar bedeutet daher bevorzugt Phenyl, o-,m- oder p-Methylphenyl, o-, m-oder p-Ethylphenyl, o-m-oder p-Propylphenyl, o-m- oder p-Isopropylphenyl, o-, m- oder p-tert. Butylphenyl, o-, m- oder p-Hydroxyphenyl-, o-, m- oder p-Methoxyphenyl, o-, m- oder p- Ethoxyphenyl-, o-, m-, p-Trifluormethylphenyl, o-, m-, p-Trifluormethoxyphenyl, o-, m- oder p-Fluorphenyl, o-, m- oder p-Chlorphenyl, o-, m- oder p-Bromphenyl, o-, m-. p-Nitrophenyl, o-,m- oder p-Aminomethylphenyl.

Het bedeutet einen gesättigten, teilweise oder vollständig ungesättigten mono- oder bicyclischen heterocylcischen Rest mit 5 bis 10 Ringgliedern, wobei 1 bis 3 N- und/ oder 1 S- oder O-Atome vorliegen können und der heterocyclische Rest ein- oder zweifach durch CN, Hal, OH, NH₂, COOH, OA, CF₃, A, NO₂, Ar oder OCF₃ substituiert sein kann.

Het ist vorzugsweise ein o-, m-, p-substituiertes Pyridyl, ein 2-, 4-, 5- oder 6-substituiertes Pyrimidyl oder ein 3-, 4-, 5- oder 6-substituiertes Pyridazyl, das bevorzugt unsubstituiert oder substituiert durch eine Methyl-, Ethyl-, Propylgruppe oder eine Methylamino-, Ethylamino- oder Propylaminogruppe ist (bezieht sich auf alle der drei genannten Heteroaromaten), sowie ein 2- substituiertes Benzimidazolyl, das unsubstituiert oder durch eine 3-Methyl-, 3-Ethyl- oder 3-Benzylgruppe substituiert ist, sowie ein 2-substituiertes Dihydroimidazolyl, Tetrahydropyrimidyl oder Tetrahydropyridyl.
Beispiele, die in Het bevorzugt enthalten sind:

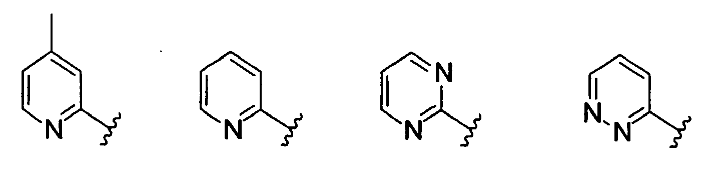

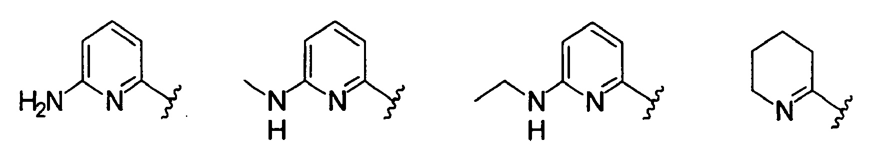

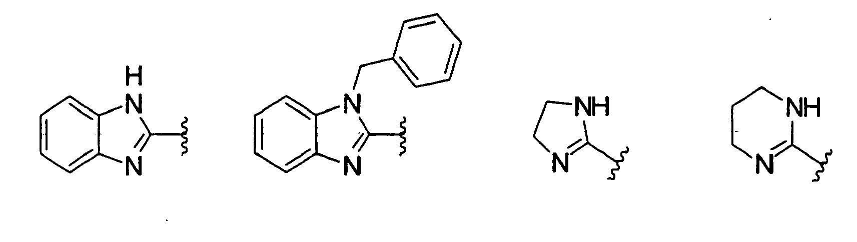

Het¹ bedeutet einen 5- oder 6-gliedrigen aromatischen Heterocyclus mit 1 bis 4 N-, O- und/ oder S-Atomen, der unsubstituiert oder ein- oder zweifach durch F, Cl, Br, A, OA oder OCF₃ substituiert sein kann.

Het¹ ist vorzugsweise ein 2,4-, 3,5-, 2,5-disubstituiertes Pyridyl oder ein 2,4-, 2,5-, 2,6-, 4,6-disubstituiertes Pyrimidyl, ein 2,4-, 2,5-disubstituiertes 1,3-Oxazolyl oder 1,3-Thiazolyl.

OA bedeutet vorzugsweise Methoxy, Ethoxy, Propoxy oder Butoxy, ferner auch Pentyloxy oder Hexyloxy.

Hal bedeutet vorzugsweise F, Cl, oder Br, aber auch I.

Die Indices n, m, o, p, q, s und t haben die im Anspruch 2 angegebene Bedeutung, sofern nicht ausdrücklich etwas anderes angegeben ist.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Besonders bevorzugt sind folgende Verbindungen der Formel I:
a) Cyclo-(Arg-Gly-Asp-D-Phe-Lys(^{ε}NH-[CO-(CH₂)₅-NH]₂Lys-(CO-C₆H₃(CH₂PO₃H₂)₂)₂))
b) Cyclo-(Arg-Gly-Asp-D-Phe-Lys(^{ε}NH-[CO-(CH₂)₅-NH]₂-(CO-C₆H₃(CH₂PO₃H₂)₂)₂))
c) Cyclo-(Arg-Gly-Asp-D-Phe-Lys(^{ε}NH-[CO-(CH₂)₅-NH]₃-Lys-(CO-C₆H₃(CH₂PO₃H₂)₂)₂))
d) Cyclo-(Arg-Gly-Asp-D-Phe-Lys(^{ε}NH-[CO-(CH₂)₅-NH]₃-(CO-C₆H₃(CH₂PO₃H₂)₂)₂))
e) Cyclo-(Arg-Gly-Asp-D-Phe-Lys(^{ε}NH-[CO-(CH₂)₅-NH]₄-Lys-(CO-C₆H₃(CH₂PO₃H₂)₂)₂))
f) Cyclo-(Arg-Gly-Asp-D-Phe-Lys(^{ε}NH-[CO-(CH₂)₅-NH]₄-(CO-C₆H₃(CH₂PO₃H₂)₂)₂))
g) Cyclo-(Arg-Gly-Asp-D-Phe-Lys(^{ε}NH-[CO-CH₂(-O-CH₂CH₂)₆-NH]₂ Lys-(CO-C₆H₃(CH₂PO₃H₂)₂)₂))
h) Cyclo-(Arg-Gly-Asp-D-Phe-Lys(^{ε}NH-[(O-CH₂CH₂)₆-NH]₂-Lys-(CO-C₆H₃(CH₂PO₃H₂)₂)₂))
i) Cyclo-(Arg-Gly-Asp-D-Phe-Lys(^{ε}NH-[CO-(CH₂)₅-NH]₂-Lys-[Lys-(CO-C₆H₃(CH₂PO₃H₂)₂)₂]₂))
j) Cyclo-(Arg-Gly-Asp-D-Phe-Lys(^{ε}NH-[CO-(CH₂)₅-NH]₃-Lys-[Lys-(CO-C₆H₃(CH₂PO₃H₂)₂)₂]₂))
k) Cyclo-(Arg-Gly-Asp-D-Phe-Lys(^{ε}NH-[CO-CH₂(-O-CH₂CH₂)₆-NH]₂-Lys-[Lys-(CO-C₆H₃(CH₂PO₃H₂)₂)₂]₂))

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Fragmentkupplung bzw. die Kupplung von Ligand an Linker erfolgt in der Regel in einem inerten Lösungsmittel, wobei ein Carbonsäurefragment (z.B. Phosphonatlinker HO-[CO-(CH₂)₅-NH]₄-Lys-[Lys-(CO-C₆H₃(CH₂-PO₃ H₂)₂)₂ mit HATU, HOAc und 2,4,6-Collidin in DMF gelöst und mit einem Aminfragment (Cyclopeptid z.B. c[R(Pbf)G(OtBu)fK])) versetzt wird.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, N-Methylpyrrolidon, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Cyclische Verbindungen können durch Cyclisierung der linearen Verbindungen hergestellt werden, wie z. B. in DE 43 10 643, in Houben-Weyl, I.c., Band 15/11, Seiten 1 bis 806 (1974) oder von S. Zimmer, E. Hoffmann, G. Jung und H. Kessler, Liebigs Ann. Chem. 1993, 497-501, beschrieben.
Die linearen Peptide können z.B. nach R.B. Merrifield, Angew. Chemie 1985, 97, 801-812, synthetisiert werden.

Offenkettige lineare Verbindungen, wie z.B. Verbindungen der Formel I können im übrigen nach üblichen Methoden der Aminosäure- und Peptidsynthese hergestellt werden, z. B. auch nach der Festphasensynthese nach Merrifield (s. auch z.B. B. F. Gysin und R. B. Merrifield, J. Am. Chem. Soc. 94, 3102 ff. (1972)).

Die Verbindungen der Formel I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OtBu)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OtBu, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol, TFA / Thioanisol oder TFA/TIPS/H₂O wird nur die Tritylgruppe von His, Asn und Gln abgespalten, wogegen sie an der Cys-Seitenkette verbleibt.
Die Pbf (Pentamethylbenzofuranyl)-gruppe wird zum Schutz von Arg eingesetzt. Die Abspaltung erfolgt z.B. mit TFA in Dichlormethan.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° C und Drucken zwischen etwa 1 und 200 bar, bevorzugt bei 10-30° C und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 10-30° C.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyl- oder Diisopropyl-ammoniumsalze, Monoethanol-, Diethanol- oder Diisopropanolammoniumsalze, Cyclohexyl-, Dicyclohexylammoniumsalze, Dibenzylethylendiammoniumsalze, weiterhin z. B. Salze mit Arginin oder Lysin.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel:
Ethylacetat/Methanol 9:1.
RZ = Retentionszeit (Minuten) bei HPLC in den folgenden Systemen:
[A]
   Säule: YMC ODS A RP 5C₁₈, 250 x 4,6 mm
   Eluent A: 0,1 % TFA in Wasser
   Eluent B: 0,1 % TFA in Acetonitril
   Fluß: 1 ml/min
   Gradient: 0 - 50 % B / 30 min.
[B]
   wie [A];
   Gradient: 5 - 50 % B / 30 min.
[C]
   wie [A];
   Gradient: 10 - 50 % B / 30 min.
Massenspektrometrie (MS): EI (Elektronenstoß-Ionisation) M⁺
   FAB (Fast Atom Bombardment) (M+H)⁺
   ESI (Elektrospray-lonisation) (M+H)⁺
DMPP-Harz steht für 4-(2',4'-Dimethoxyphenyl-hydroxymethyl)phenoxy-Harz, welches z.B. die Synthese von seitenkettengeschützten Peptiden erlaubt, TCP-Harz bedeutet Tritylchlorid-Polystyrol-Harz.

Die nachfolgenden Beispiele beschreiben zum einen die Fragmentkupplung sowie die Spaltung von Phosphonestem und zum anderen die Synthese ausgewählter Cyclopeptid-Derivate mit Phosphonatlinker. Anhand der Beispiele 7 bis 9 wird das Verfahren zur Beschichtung der verschiedenen Formkörper aus Metall oder Knochenersatzmaterialien näher erläutert.

### Beispiel 1: Fragmentkupplung in Lösung

0.2 mmol Carbonsäurefragment (z.B. Phosphonatlinker HO-[CO-(CH₂)₅-NH]₄-Lys-(CO-C₆H₃(CH₂PO₃H₋₂)₂)₂), 0.98 eq HATU, 1.1 eq HOAc und 10 eq 2,4,6-Collidin werden in 2 mL DMF gelöst. Nach 1.5 h wird 1 eq Aminfragment (z.B. Cyclopeptid c[R(Pbf)G(OtBu)fK]) zugegeben. Man läßt 24 h bei Raumtemperatur rühren und reinigt das Produkt mittels präparativer HPLC.

### Beispiel 2: Spaltung von Phosphonestem bei den Phosphonatlinkern

Die Spaltung der Phosphonatestergruppen erfolgt gleichzeitig mit der Abspaltung der Seitenkettenschutzgruppen des Peptids oder Peptidmimetikas mit 90% TFA, 5% H₂O und 5 % TIPS. Nach 4 h wird das Lösungsmittel abgezogen, der Rückstand in Essigsäure aufgenommen und in kaltem Diethylether gefällt. Der Niederschlag wird abgetrennt und aus H₂O lyophilisiert.

### Beispiel 3: Synthese der Phosphonatlinker

Die Phosphonatlinker wurden in einer Festphasenpeptidsynthese nach der Fmoc-Strategie synthetisiert (siehe G.B. Fields, R. L. Nobie, Int. J. Pept. Protein Res. 1990, 35, 161-214).
Als letzter Baustein wurde 5-Carboxy-m-xylen-bisphosphonsäure-tetrabenzylester gekuppelt.

Synthese von 5- Carboxy-m-xylen-bisphosphonsäure-tetrabenzylester:

### 3,5-Bis(brommethyl)benzoesäuremethylester

30 mmol Methyl-3,5-(bismethyl)benzoat (5.0 g) werden in 50 ml CCl₄ gelöst. Nach der Zugabe von 2 eq N-Bromsuccinimid (10.6 g) und 150 mg Benzoylperoxide wird die Mischung 3 h refluxiert. Die erkaltete Mischung wird filtriert und das Lösungsmittel abgezogen. Das Produkt fällt als Öl an (10.5 g), welches durch Überschichten mit 100 ml Hexan zur Kristallisation gebracht werden kann. Es werden 3.3 g blassgelber Feststoff erhalten.

### 5-Methoxycarbonyl-m-xylen-bisphosphonsäure-tetrabenzylester

4.7 mmol 3,5 Bis(brommethyl)benzoeäsuremethylester (1.5 g) werden in 3 eq Tribenzylphosphit (4.9g) suspendiert. Die Mischung wird für 3 h im 140 °C heißem Ölbad erhitzt, während das enstehende Benzylbromid im Hochvakuum abgezogen wird. Der Rückstand wird nach dem Erkalten an 250 g Kieselgel mit Ethylacetat als Eluent chromatografisch getrennt. Das Produkt fällt als Öl an (1.76 g).

### 5-Carboxy-m-xylen-bisphosphonsäure-tetrabenzylester

1.5 mmol 5-Methoxycarbonyl-m-xylen-bisphosphonsäure-tetrabenzylester (1.0 g) werden in Methanol und Wasser 2:1 gelöst und mit 1.5 eq Lithiumhydroxid (53 mg) für vier Tage bei Raumtemperatur gerührt. Anschließend wird der pH-Wert der Lösung mit 1 N Salzsäure auf 2.5 eingestellt und das Methanol abgezogen. Das Produkt wird mit Ethylacetat extrahiert. Nach Abzug des Lösungsmittels werden 0.97 g Öl erhalten.

Ti- bzw. TiAl₆V₄-Formkörper mit einem Durchmesser von 10 mm und einer Höhe von 1-2 mm werden gereinigt.

Die Formkörper werden in 48-wells (Costar, "non-tissue culture treated" Art. Nr. 3574) transferiert. Zur Anbindung der bioaktiven, zelladhäsionsvermittelnden Moleküle B (wobei B ein Cyclopeptid, Peptidmimetikum oder lineares Peptid gemäß Anspruch 2 sein kann) an die vorbereiteten Formkörper werden Moleküle B -enthaltende Stammlösungen ("B-Lösungen") in einer Endkonzentration von 1 mM in einem wäßrigen Puffer hergestellt. Anschließend werden durch Verdünnung mit Puffer Konzentrationsreihen mit den "B-Lösungen"-Endkonzentrationen von jeweils 1 nM, 10 nM, 100 nM, 1 µM, 10 µM und 100 µM hergestellt. Die Formkörper werden mit je 250 µl der jeweiligen B-Lösungen überschichtet und anschließend für 18-24 Stunden bei Raumtemperatur inkubiert. Zur Entfernung ungebundener B-Moleküle wurden die Proben dreimal mit Puffer gewaschen und über Nacht in Puffer gelagert.

Durch Zugabe von je 250 µl/ Formkörper einer 5%igen BSA (Bovine Serum Albumine)-Lösung, pH 7.4, anschließender Inkubation für 2 Stunden bei Raumtemperatur und einmaligem Waschen mit Puffer werden unspezifische Zellbindungsstellen blockiert.
Als Negativkontrollen fungieren Ti- bzw. TiAl₆V₄-Formkörper, die anstelle von B-Lösungen mit entsprechenden Pufferlösungen (Tris-HCI 10 mM, pH 8.7; Tris-HClO₄ 10 mM, pH 8.7; PBS, pH 7.4) behandelt werden.
Der Grad der erhaltenen Beschichtungen auf den Formkörpern wird analytisch bewertet und die biologische Wirksamkeit mittels eines Zelladhäsionstests in vitro bestimmt.

### Beispiel 4:

Formkörper auf Calciumphosphatbasis werden gereinigt.

Zur Beschichtung der Formkörper mit der "B-Lösung" wird wie unter Beispiel 3 beschrieben verfahren.

Der Grad der erhaltenen Beschichtung auf den Formkörpern wird analytisch bewertet und die biologische Wirksamkeit mittels eines Zelladhäsionstests in vitro bestimmt.

### Beispiel für ELISA Test:

Mittels eines RGD spezifischen Antikörpers lässt sich die Menge an gebunden Peptid auf der Oberfläche ermitteln.

### Beispiel für Zelladhäsionstest:

Es wurde die Adhäsion von Maus-MC3T3 E1-Osteoblastenkulturen in vitro an RGD-Peptid-beschichteten Titanoberflächen untersucht. Dabei wurden 50.000 Zellen/cm² angesät und nach einstündiger Inkubation in serumfreiem Medium bei 37° C / 95 % Luftfeuchte der Anteil adhärierter Zellen bestimmt.

### Zellanhaftungsrate [%] = adherierte Zellen / angesäte Zellen x 100

### Peptid: Zellanhaftungsrate [%]

### Ergebnisse des ELISA Test

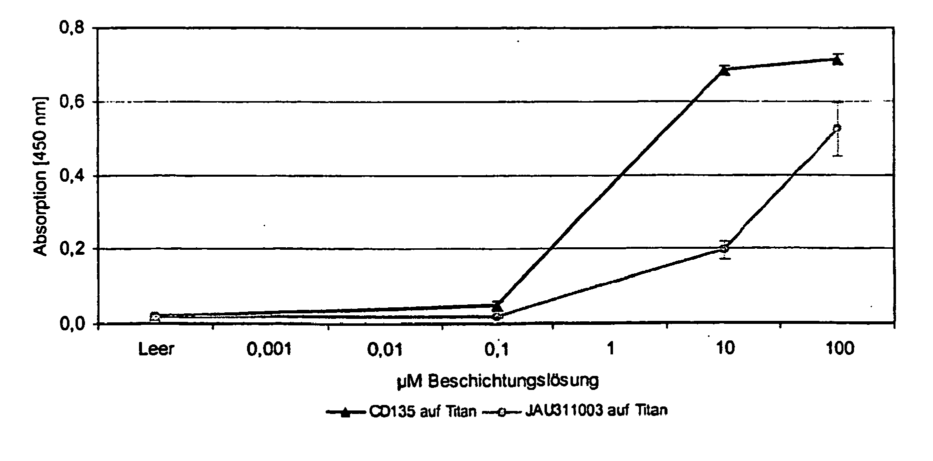

### Ergebnisse der Zelladhäsion

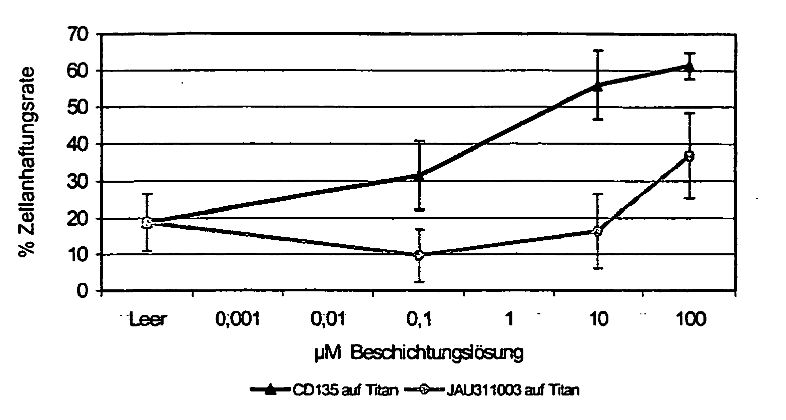

Die in der Abbildung dargestellten Abkürzungen bedeuten folgendes: Bei CD135 handelt es sich um Cyclo-(Arg-Gly-Asp-D-Phe-Lys(^{ε}NH-[CO-(CH₂)₅-NH]₃-(CO-C₆H₃(CH₂PO₃H₂)₂) bei JAU311003 um Cyclo-(Arg-Gly-Asp-D-Phe-Lys(^{ε}NH-[CO-(CH₂)₅-NH]₃(CO-C₆H₃(CH₂PO₃H₂)₂)₂)).
Im ELISA ist zu erkennen, dass im Fall der Verbindung CD 135 mehr Peptid auf der Oberfläche gebunden wird, was bei der Zelladhäsion zu einer höheren Zellanhaftungsrate bei allen gemessen Konzentrationen führt.
Die Zelladhäsion zu Beginn (Leer) entspricht der Zelladhäsion bei unbeschichteten Titanplättchen. Durch die Beschichtung der Plättchen mit einer 100 µM Beschichtungslösung lässt sich die Zelladhäsion im Vergleich zu unbeschichteten Plättchen verdreifachen.

## Patentansprüche

1. Verfahren zur Herstellung einer Verbindung der Formel I
B-Q-X₁ I,
worin
B ein bioaktives, zelladhäsionsvermittelndes Molekül ist,
Q fehlt oder ein organisches Spacer-Molekül ist und
X₁ ein Ankermolekül ist, ausgewählt aus der Gruppe
-W (i)
-V-W (ii)
-V-[V-W₂]₂ (iii)
oder
-V-[V-(V-W₂)₂]₂ (iv),
wobei W 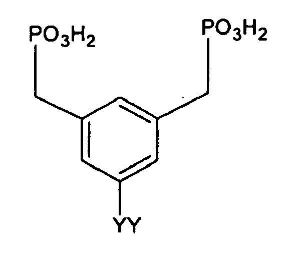
und V Lys, Asp oder Glu
bedeutet, YY eine Carboxylgruppe ist, wobei eine freie Aminogruppe der Gruppe B mit einer freien Carboxylgruppe des Spacer-Moleküls Q oder des Ankermoleküls X₁, bzw. eine freie Aminogruppe des Restes Q mit einer freien Carboxylgruppe des Restes X₁ peptidartig verknüpft ist,
oder deren Salze,
**dadurch gekennzeichnet,**
**dass** man das bioaktive Molekül B, welches mit Schutzgruppen versehen sein kann, und das mit Schutzgruppen und Phosphonestergruppen versehene Spacer-Anker-Molekül (Q-X₁) bzw. Anker-Molekül (X₁) peptidisch miteinander verknüpft und anschließend die Schutzgruppen gleichzeitig mit den Phosphonestergruppen abspaltet; und/oder dass man eine basische oder saure Verbindung der Formel I durch Behandeln mit einer Säure oder Base in eines ihrer Salze überführt, wobei die Phosphonestergruppen 5-Carboxy-m-xylen-bisphosphonsäure-tetrabenzylester-Gruppen sind.

2. Verfahren nach Anspruch 1, **dadurch gekennzeichnet, dass** die Gruppe B ausgewählt ist aus der Gruppe
cyclo-(Arg-Gly-Asp-Z1) (iv) 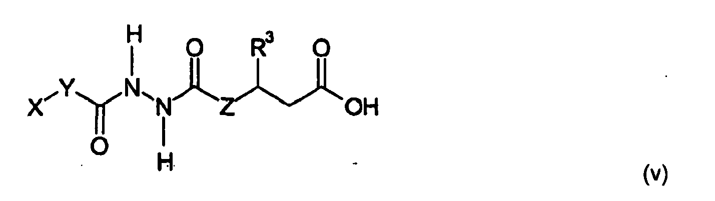
und
Thr-Trp-Tyr-Lys-Ile-Ala-Phe-Gln-Arg-Asn-Arg-Lys (vi)
Trp-Tyr-Lys-Ile-Ala-Phe-Gln-Arg-Asn-Arg-Lys (vii)
Tyr-Lys-Ile-Ala-Phe-Gln-Arg-Asn-Arg-Lys (viii)
Thr-Trp-Tyr-Lys-Ile-Ala-Phe-Gln-Arg-Asn-Arg (ix)
Thr-Trp-Tyr-Lys-Ile-Ala-Phe-Gln-Arg-Asn (x)
Thr-Trp-Tyr-Lys-Ile-Ala-Phe-Gln-Arg (xi),
worin
Z₁ jeweils unabhängig voneinander einen Aminosäurerest oder einen Di- oder Tripeptidrest bedeutet, wobei die Aminosäuren unabhängig voneinander ausgewählt sind aus einer Gruppe bestehend aus Ala, Asn, Asp, Arg, Cys, Gln,
Glu, Gly, His, Homo-Phe, Ile, Leu, Lys, Met, Orn, Phe, Phg, Pro, Ser, Thr, Trp, Tyr, Val,
worin für (v)
X H₂N-C(=NH)-NH, Het-NH-, H₂N-C(=NH)-, A-C(=NH)-NH oder Het-
Y -(CH₂)ₙ- oder 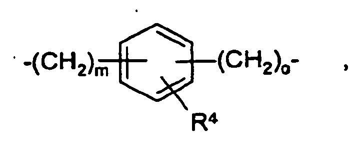 -(CH₂)ₛ-CH(R⁴)-(CH₂)ₜ- oder -(CH₂)ₚ-Het¹-(CH₂)_{q}
Z N-R² oder CH-R²
R² H oder Alkyl mit 1 bis 4 C-Atomen
R³ H, Ar, Het oder A
R⁴ H, A, Ar, OH,OA, OAr, Arylalkyl, Hal ,CN, NO₂, CF₃ oder OCF₃,
A COOH, NH₂ oder Alkyl mit 1-6 C-Atomen, unsubstituiert oder substituiert mit COOH oder NH₂
Ar unsubstituiertes oder ein-, zwei- oder dreifach durch A, OH, OA, CF₃, OCF₃, CN, NO₂ oder Hal substituiertes Phenyl, welches durch ein ein-, zwei oder dreifach durch A, OH, OA, NH₂, OCF₃, CN, NO₂ oder Hal substituiertes Phenyl in der Art substituiert sein kann, dass ein unsubstituiertes oder substituiertes Biphenyl entsteht,
Hal F, Cl, Br oder I,
Het einen gesättigten, teilweise oder vollständig ungesättigten mono- oder bicyclischen heterocyclischen Rest mit 5 bis 10 Ringgliedern, wobei 1 bis 3 N- und/oder 1 S- oder O-Atome vorliegen können und der heterocyclische Rest ein- oder zweifach durch CN, Hal, OH , NH₂, COOH, OA, CF₃, A, NO₂, Ar oder OCF₃ substituiert sein kann,
Het¹ einen 5- oder 6-gliedrigen aromatischen Heterocyclus mit 1 bis 4 N- und / oder S-Atomen, der unsubstituiert oder ein- oder zweifach durch F, Cl, Br, A, OA oder OCF₃ substituiert sein kann,
n 4, 5 oder 6,
m, o, p, q 0, 1 oder 2,
s, t 0,1, 2, 3, 4 oder 5
ist.

3. Verfahren nach Anspruch 1 oder 2, **dadurch gekennzeichnet, dass** Q ausgewählt ist aus der Gruppe
[CO-(CH₂)ₓ-NH-]ₘ (xii)
[CO-CH₂(-O-CH₂CH₂)_{y}-NH-]ₘ (xiii)
[CO-(CH₂)_{z}-CO-] (xiv)
[NH-(CH₂)_{z}-NH-] (xv)
[CO-CH₂-(OCH₂CH₂)_{y}-O-CH₂-CO-] (xvi)
[NH-CH₂CH₂-(OCH₂CH₂)_{y}-NH-] (xvii)
sowie deren Kombination,
worin
m jeweils unabhängig voneinander 1- 20
x 1- 12
y 1- 50 und
z 1-12 ist.

4. Verfahren nach einem der vorhergehenden Ansprüche, **dadurch gekennzeichnet, dass** Q ausgewählt ist aus der Gruppe
[CO-(CH₂)ₓ-NH-]ₘ, (xviii)
[CO-CH₂(-O-CH₂CH₂)_{y}-NH-]ₘ (xix)
[CO-(CH₂)_{z}-CO-] (xx)
[NH-(CH₂)_{z}-NH-] (xxi)
[CO-CH₂-(OCH₂CH₂)_{y}-O-CH₂-CO-] (xxii)
[NH-CH₂CH₂-(OCH₂CH₂)_{y}-NH-] (xxiii)
sowie deren Kombination,
worin
m jeweils unabhängig voneinander 1 bis 8,
x 1- 5,
y 1- 6 und
z 1- 6 ist.

5. Verfahren nach einem der vorhergehenden Ansprüche, **dadurch gekennzeichnet, dass** die Verbindung der Formel I eine der folgenden Verbindungen ist:
a) Cyclo-(Arg-Gly-Asp-D-Phe-LysNH-[CO-(CH₂)₅-NH]₂-Lys-(CO-C₆H₃(CH₂PO₃H₂)₂)₂))
b) Cyclo-(Arg-Gly-Asp-D-Phe-Lys(^{ε}NH-JCO-(CH₂)₅-NH)₂-(CO-C₆H₃(CH₂PO₃H₂)₂)₂))
c) Cyclo-(Arg-Gly-Asp-D-Phe-Lys(^{ε}NH-[CO-(CH₂)₅-NH]₃-Lys-(CO-C₆H₃(CH₂PO₃H₂)₂)₂))
d) Cyclo-(Arg-Gly-Asp-D-Phe-Lys(^{ε}NH-[(CO-(CH₂)₅-NH]₃-(CO-C₆H₃(CH₂PO₃H₂)₂)₂))
e) Cyclo-(Arg-Gly-Asp-D-Phe-Lys(^{ε}NH-[CO-(CH₂)₅-NH]₄-Lys-(CO-C₆H₃(CH₂PO₃H₂)₂)₂))
f) Cyclo-(Arg-Gly-Asp-D-Phe-Lys(^{ε}NH-[CO-(CH₂)₅-NH₄](CO-C₆H₃(CH₂PO₃H₂)₂)₂))
g) Cyclo-(Arg-Gly-Asp-D-Phe-Lys(^{ε}NH-[CO-CH₂(-O-CH₂CH₂)₆-NH]₂-Lys-(CO-C₆H₃(CH₂PO₃H₂)₂)₂))
h) Cyclo-(Arg-Gly-Asp-D-Phe-Lys(^{ε}NH-[(-O-CH₂CH₂)₆-NH]₂-Lys-(CO-C₆H₃(CH₂PO₃H₂)₂)₂))
i) Cyclo-(Arg-Gly-Asp-D-Phe-Lys(^{ε}NH-[CO-(CH₂)₅-NH]₂-Lys-[Lys-(CO-C₆H₃(CH₂PO₃H₂)₂)₂]₂))
j) Cyclo-(Arg-Gly-Asp-D-Phe-Lys(^{ε}NH-[CO-(CH₂)₅-NH]₃-Lys-[Lys-(CO-C₆H₃(CH₂PO₃H₂)₂)₂]₂))
k) Cyclo-(Arg-Gly-Asp-D-Phe-Lys(^{ε}NH-[CO-CH₂(O-CH₂CH₂)₆-NH]₂-Lys-[Lys-(CO-C₆H₃(CH₂PO₃H₂)₂)₂]₂))

6. Verfahren zur Herstellung beschichteter Implantate geeignet für humane und tierische Organe, bestehend aus einer Trägermatrix und einer diese Matrix umhüllenden Schicht eines bioaktiven, zelladhäsionsvermittelnden Moleküls, **dadurch gekennzeichnet, dass** die umhüllende Schicht aus einer Verbindung, die mittels eines Verfahrens nach einem der Ansprüche 1 bis 5 hergestellt ist, gebildet wird, wobei zwischen Trägermatrix und dieser Verbindung eine ionische oder adsorptive Bindung ausgebildet wird.

7. Verfahren nach Anspruch 6, **dadurch gekennzeichnet, dass** die Trägermatrix und/oder deren Oberfläche ein Metall oder ein Metalloxid ist.

8. Verfahren nach Anspruch 6, **dadurch gekennzeichnet, dass** die Trägermatrix und/oder deren Oberfläche ein Knochen- oder Zahnersatzmaterial ist.

9. Verfahren nach Anspruch 8, **dadurch gekennzeichnet, dass** das Knochen- oder Zahnersatzmaterial aus Calciumphosphat-Mischungen besteht.

## Claims

1. Process for preparing a compound of the formula I
B-Q-X₁ I,
in which
B is a bioactive, cell adhesion-mediating molecule,
Q is absent or is an organic spacer molecule, and
X₁ is an anchor molecule selected from the group
**-W** **(i)**
**-V-W** **(ii)**
**-V-[V-W₂]₂** **(iii)**
or
**-V-[V-(V-W₂)₂]₂** **(iv),**
where w is 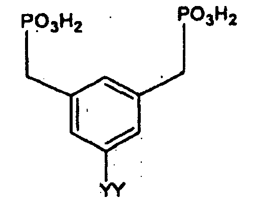
and V is Lys, Asp or Glu,
YY is a carboxyl group, where a free amino group of the group B is linked to a free carboxyl group of the spacer molecule Q or of the anchor molecule X₁, or a free amino group of the residue Q is linked to a free carboxyl group of the residue X₁, like peptides,
or the salts thereof,
**characterized in that**
the bioactive molecule B, which can be provided with protective groups, and the spacer-anchor molecule (Q-X₁) or anchor molecule (X₁), which is provided with protective groups and phosphonic ester groups, are linked to one another in peptide fashion, and then the protective groups are eliminated at the same time as the phosphonic ester groups, and/or **in that** a basic or acidic compound of the formula I is converted into one of its salts by treating with an acid or base, where the phosphonic ester groups are 5-carboxy-m-xylenebisphosphonic acid tetrabenzyl ester groups.

2. Process according to Claim 1, **characterized in that** the group B is selected from the group
**cyclo-(Arg-Gly-Asp-Z1)** **(iv)** 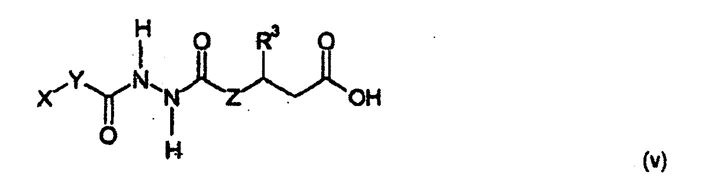 and
**Thr-Trp-Tyr-Lys-Ile-Ala-Phe-Gln-Arg-Asn-Arg-Lys** **(vi)**
**Trp-Tyr-Lys-Ile-Ala-Phe-Gln-Arg-Asn-Arg-Lys** **(vii)**
**Tyr-Lys-Ile-Ala-Phe-Gln-Arg-Asn-Arg-Lys** **(viii)**
**Thr-Trp-Tyr-Lys-Ile-Ala-Phe-Gln-Arg-Asn-Arg** **(ix)**
**Thr-Trp-Tyr-Lys-Ile-Ala-Phe-Gln-Arg-Asn** **(x)**
**Thr-Trp-Tyr-Lys-Ile-Ala-Phe-Gln-Arg** **(xi),**
in which
Z₁ is in each case independently of one another an amino acid residue or a di- or tripeptide residue, where the amino acids are selected independently of one another from a group consisting of Ala, Asn, Asp, Arg, Cys, Gin, Glu, Gly, His, homo-Phe, Ile, Leu, Lys, Met, Om, Phe, Phg, Pro, Ser, Thr, Trp, Tyr, Val,
in which for (v)
x is H₂N-C(=NH)-NH, Het-NH-, H₂N-C (=NH) -, A-C(=NH)-NH or Het-,
Y is -(CH₂)ₙ- or 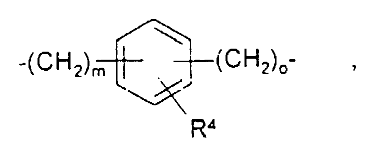 -(CH₂)ₛ-CH(R⁴)-(CH₂)ₜ- or -(CH₂)ₚ-Het¹-(CH₂)_{q}-,
Z is N-R² or CH-R²,
R² is H or alkyl having 1 to 4 C atoms,
R³ is H, Ar, Het or A,
R⁴ is H, A, Ar, OH, OA, OAr, arylalkyl, Hal, CN, NO₂, CF₃ or OCF₃,
A is COOH, NH₂ or alkyl having 1-6 C atoms, which is unsubstituted or substituted by COOH or NH₂,
Ar is phenyl which is unsubstituted or mono-, di- or trisubstituted by A, OH, OA, CF₃, OCF₃, CN, NO₂ or Hal, which can be substituted by a phenyl which is mono-, di- or trisubstituted by A, OH, OA, NH₂, OCF₃, CN, NO₂ or Hal in such a way that an unsubstituted or substituted biphenyl results,
Hal is F, Cl, Br or I,
Het is a saturated, partly or completely unsaturated mono- or bicyclic heterocyclic radical having 5 to 10 ring members, where 1 to 3 N and/or 1 S or O atom(s) can be present and the heterocyclic radical can be mono- or disubstituted by CN, Hal, OH, NH₂, COOH, OA, CF₃ , A, NO₂, Ar or OCF₃ ,
Het¹ is a 5- or 6-membered aromatic heterocycle having 1 to 4 N and/or S atoms, which can be unsubstituted or mono- or disubstituted by F, Cl, Br, A, OA or OCF₃,
n is 4, 5 or 6,
m, o, p, q are 0, 1 or 2,
s, t are 0, 1, 2, 3, 4 or 5.

3. Process according to Claim 1 or 2, **characterized in that** Q is selected from the group
**[CO-(CH₂)ₓ-NH-]ₘ** **(xii)**
**[CO-CH₂(-O-CH₂CH₂)_{y}-NH-]ₘ** **(xiii)**
**[CO-(CH₂)_{z}-CO-]** **(xiv)**
**(NH-(CH₂)_{z}-NH-)** **(xv)**
**[CO-CH₂-(OCH₂CH₂)_{y}-O-CH₂-CO-]** **(xvi)**
**[NH-CH₂CH₂-(OCH₂CH₂)_{y}-NH-]** **(xvii)**
and their combination,
in which
m is in each case independently of one another 1-20
x is 1-12
y is 1-50 and
z is 1-12.

4. Process according to any of the preceding claims, **characterized in that** Q is selected from the group
**[CO-(CH₂)ₓ-NH-]ₘ** **(xviii)**
**[CO-CH₂(-O-CH₂CH₂)_{y}-NH-]ₘ** **(xix)**
**[CO-(CH₂)_{z}-CO-]** **(xx)**
**[NH-(CH₂)_{z}-NH-]** **(xxi)**
**[CO-CH₂-(OCH₂CH₂)_{y}-O-CH₂-CO-]** **(xxii)**
**[NH-CH₂CH₂-(OCH₂CH₂)_{y}-NH-]** **(xxiii)**
and their combination,
in which
m is in each case independently of one another 1 to 8,
x is 1-5,
y is 1-6 and
z is 1-6.

5. Process according to any of the preceding claims, **characterized in that** the compound of the formula I is one of the following compounds:
**a) Cyclo-(Arg-Gly-Asp-D-Phe-LysNH-[CO-(CH₂)₃-NH]₂-Lys-(CO-C₆H₃(CH₂PO₃H₂)₂)₂))**
**b) Cyclo-(Arg-Gly-Asp-O-Phe-Lys(^{ε}NH-[CO-(CH₂)₅-NH]₂(CO-C₆H₃(CH₂PO₃H₂)₂)₂))**
**c) Cyclo-(Arg-Gly-Asp-D-Phe-Lys(^{ε}NH-[CO-(CH₂)₅-NH]₃-Lys-(CO-C₆H₃(CH₂PO₃H₂)₂)₂))**
**d) Cyclo-(Arg-Gly-Asp-D-Phe-Lys(^{ε}NH-[CO-(CH₂)₅-NH]₃-(CO-C₈H₃(CH₂PO₃H₂)₂)₂))**
**e) Cyclo-(Arg-Gly-Asp-D-Phe-Lys(^{ε}NH-[CO-(CH₂)₅-NH]₄-Lys-(CO-C₆H₃)(CH₂PO₃H₂)₂)₂))**
**f) Cyclo-(Arg-Gly-Asp-D-Phe-lys(^{ε}NH-[CO-(CH₂)₅-NH]₄-(CO-C₆H₃(CH₂PO₃H₂)₂)₂))**
**g) Cyclo-(Arg-Gly-Asp-D-Phe-Lys(^{ε}NH-[CO-CH₂)(-O-CH₂CH₂)₆-NH]₂-Lys-(CO-C₆H₃(CH₂PO₃H₂)₂)₂))**
**h) Cyclo-(Arg-Gly-Asp-D-Phe-Lys(^{ε}NH-[(-O-CH₂CH₂)₆-NH]₂-Lys-(CO-C₆H₃(CH₂PO₃H₂)₂)₂))**
**i) Cyclo-(Arg-Gly-Asp-D-Phe-Lys(^{ε}NH-[CO-(CH₂)₅-NH]₂Lys-[Lys-(CO-C₆H₃(CH₂PO₃H₂)₂)₂)₂))**
**j) Cyclo-Arg-Gly-Asp-D-Phe-Lys(^{ε}NH-[CO-(CH₂)₅₋NH]₃Lys-[Lys-(CO-C₆H₃(CH₂PO₃H₂)₂)₂)₂))**
**k) Cyclo-(Arg-Gly-Asp-D-Phe-Lys(^{ε}NH[CO-CH₂(O-CH₂CH₂)₆-NH]₂-Lys-(CO-C₆H₃(CH₂PO₃H₂)₂)₂]₂))**

6. Process for producing coated implants suitable for human and animal organs, consisting of a carrier matrix and a layer of a bioactive, cell adhesion-mediating molecule surrounding this matrix, **characterized in that** the surrounding layer is formed from a compound which is prepared by means of a process according to any of Claims 1 to 5, an ionic or adsorptive bond being formed between carrier matrix and this compound.

7. Process according to Claim 6, **characterized in that** the carrier matrix and/or its surface is a metal or a metal oxide.

8. Process according to Claim 6, **characterized in that** the carrier matrix and/or its surface is a bone or tooth substitute material.

9. Process according to Claim 8, **characterized in that** the bone or tooth substitute material consists of calcium phosphate mixtures.

## Revendications

1. Procédé de préparation d'un composé de formule I
B-Q-X₁ I,
dans laquelle
B est une molécule bioactive, médiatrice de l'adhésion cellulaire,
Q est absent ou est une molécule d'espaceur organique, et
X₁ est une molécule d'ancrage, choisie parmi le groupe
-W (i)
-V-W (ii)
-V- [V-W₂]₂ (iii)
ou
-V- [V- ( V-W₂)₂]₂ (iv),
où W représente 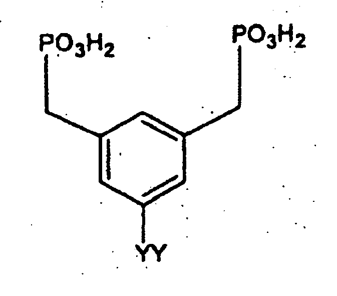
et V représente Lys, Asp ou Glu,
YY est un groupe carboxyle, où un groupe amino libre du groupe B est lié de façon analogue à un peptide à un groupe carboxyle libre de la molécule d'espaceur Q ou de la molécule d'ancrage X₁, ou un groupe amino libre du reste Q est lié de façon analogue à un peptide à un groupe carboxyle libre du reste X₁,
ou de sels de celui-ci,
**caractérisé en ce que**
la molécule bioactive B, qui peut être pourvue de groupes protecteurs, et la molécule d'espaceur-ancrage (Q-X₁) ou la molécule d'ancrage (X₁), pourvue de groupes protecteurs et de groupes ester phosphonique, sont liées l'une à l'autre de façon peptidique, et les groupes protecteurs sont ensuite éliminés en même temps que les groupes ester phosphonique, et/ou **en ce qu'**un composé acide ou basique de formule I est converti en l'un de ses sels par traitement avec un acide ou une base, les groupes ester phosphonique étant des groupes ester tétrabenzylique de l'acide 5-carboxy-m-xylène-bisphosphonique.

2. Procédé selon la revendication 1, **caractérisé en ce que** le groupe B est choisi parmi le groupe
cyclo-(Arg-Gly-Asp-Z1) (iv) 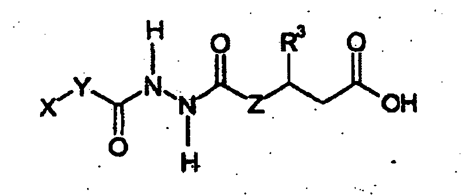
et
Thr-Trp-Tyr-Lys-Ile-Ala-Phe-Gln-Arg-Asn-Arg-Lys (vi)
Trp-Tyr-Lys-Ile-Ala-Phe-Gln-Arg-Asn-Arg-Lys (vii)
Tyr-Lys-Ile-Ala-Phe-Gln-Arg-Asn-Arg-Lys (viii)
Thr-Trp-Tyr-Lys-Ile-Ala-Phe-Gln-Arg-Asn-Arg (ix)
Thr-Trp-Tyr-Lys-Ile-Ala-Phe-Gln-Arg-Asn (x)
Thr-Trp-Tyr-Lys-Ile-Ala-Phe-Gln-Arg (xi),
où
Z₁ représente dans chaque cas, indépendamment l'un de l'autre, un radical acide aminé ou un radical di- ou tripeptidique, où les acides aminés sont choisis, indépendamment les uns des autres, parmi un groupe constitué de Ala, Asn, Asp, Arg, Cys, Gln, Glu, Gly, His, Homo-Phe, Ile, Leu, Lys, Met, Orn, Phe, Phg, Pro, Ser, Thr, Trp, Tyr, Val,
où pour (v)
x représente un groupe H₂N-C(=NH)-NH, un groupe Het-NH-, un groupe H₂N-C (=NH) -, un groupe A-C(=NH)-NH ou un groupe Het-,
Y représente un groupe -(CH₂)ₙ- ou un groupe 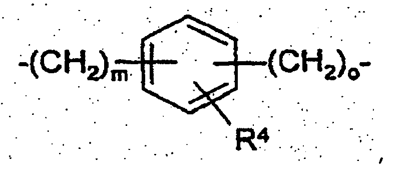 un groupe -(CH₂)ₛ-CH(R⁴)-(CH₂)ₜ- ou un groupe-(CH₂)ₚ-Het¹-(CH₂)_{q}-,
Z représente un groupe N-R² ou un groupe CH-R²,
R² représente un atome d'hydrogène ou un groupe alkyle renfermant de 1 à 4 atomes de carbone,
R³ représente un atome d'hydrogène, un groupe Ar, un groupe Het ou un groupe A,
R⁴ représente un atome d'hydrogène, un groupe A, un groupe Ar, un groupe OH, un groupe OA, un groupe OAr, un groupe arylalkyle, un groupe Hal, un groupe CN, un groupe NO₂, un groupe CF₃ ou un groupe OCF₃,
A représente un groupe COOH, un groupe NH₂ ou un groupe alkyle renfermant de 1 à 6 atomes de carbone, non substitué ou substitué par un groupe COOH ou un groupe NH₂,
Ar représente un groupe phényle non substitué ou mono-, bi- ou tri-substitué par un groupe A, un groupe OH, un groupe OA, un groupe CF₃, un groupe OCF₃, un groupe CN, un groupe NO₂ ou un groupe Hal, qui peut être substitué par un groupe phényle mono-, bi- ou tri-substitué par un groupe A, un groupe OH, un groupe OA, un groupe NH₂, un groupe OCF₃ , un groupe CN, un groupe NO₂ ou un groupe Hal de telle sorte qu'il en résulte un groupe biphényle non substitué ou substitué,
Hal représente un atome de fluor, un atome de chlore, un atome de brome ou un atome d'iode,
Het représente un reste hétérocyclique, monocyclique ou bicyclique, saturé, partiellement ou totalement insaturé, renfermant de 5 à 10 chaînons du noyau, où de 1 à 3 atomes d'azote et/ou 1 atome de soufre ou d'oxygène peuvent être présents et le reste hétérocyclique peut être mono- ou bi-substitué par un groupe CN, un groupe Hal, un groupe OH, un groupe NH₂, un groupe COOH, un groupe OA, un groupe CF₃, un groupe A, un groupe NO₂, un groupe Ar ou un groupe OCF₃,
Het¹ représente un hétérocycle aromatique à 5 ou 6 chaînons, renfermant de 1 à 4 atomes d'azote et/ou de soufre, qui peut être non substitué ou mono- ou bi-substitué par un atome de fluor, un atome de chlore, un atome de brome, un groupe A, un groupe OA ou un groupe OCF₃,
n vaut 4, 5 ou 6,
m, o, p, q valent 0, 1 ou 2,
s, t valent 0, 1, 2, 3, 4 ou 5.

3. Procédé selon la revendication 1 ou 2, **caractérisé en ce que** Q est choisi parmi le groupe
[CO-(CH₂)ₓ-NH-]ₘ (xii)
[CO-CH₂(-O-CH₂CH₂)_{y}-NH-]ₘ (xiii)
[CO-(CH₂)_{z}-CO-] (xiv)
[NH-(CH₂)_{z}-NH-] (xv)
[CO-CH₂-(OCH₂CH₂)_{y}-O-CH₂-CO-] (xvi)
[NH-CH₂CH₂-(OCH₂CH₂)_{y}-NH-] (xvii)
ainsi que leurs combinaisons,
où
m vaut dans chaque cas, indépendamment l'un de l'autre, de 1 à 20,
x vaut de 1 à 12,
y vaut de 1 à 50, et
z vaut de 1 à 12.

4. Procédé selon l'une quelconque des revendications précédentes, **caractérisé en ce que** Q est choisi parmi le groupe
[CO-(CH₂)ₓ-NH-]ₘ (xviii)
[CO-CH₂(-O-CH₂CH₂)_{y}-NH-]ₘ (xix)
[ CO- (CH₂) _{z}-CO-] (xx)
[NH-(CH₂)_{z}-NH-] (xxi)
[CO-CH₂- (OCH₂CH₂)_{y}-O-CH₂-CO-] (xxii)
[NH-CH₂CH₂- (OCH₂CH₂) _{y}-NH-] (xxiii)
ainsi que leurs combinaisons,
où
m vaut dans chaque cas, indépendamment l'un de l'autre, de 1 à 8,
x vaut de 1 à 5,
y vaut de 1 à 6, et
z vaut de 1 à 6.

5. Procédé selon l'une quelconque des revendications précédentes, **caractérisé en ce que** le composé de formule I est l'un des composés suivantes :
a) cyclo-(Arg-Gly-Asp-D-Phe-LysNH-[CO-(CH₂)₅-NH]₂-Lys-(CO-C₆H₃(CH₂PO₃H₂)₂)₂))
b) cyclo-(Arg-Gly-Asp-D-Phe-Lys(^{ε}NH-[CO-(CH₂)₅-NH]₂-(CO-C₆H₃(CH₂PO₃H₂)₂)₂))
c) cyclo- (Arg-Gly-Asp-D-Phe-Lys (^{ε}NH- [CO- (CH₂)₅-NH]₃-Lys-(CO-C₆H₃((CH₂PO₃H₂)₂)₂))
d) cyclo-(Arg-Gly-Asp-D-Phe-Lys(^{ε}NH-[(CO-(CH₂)₅-NH]₃-(CO-C₆H₃(CH₂PO₃H₂)₂)₂))
e) cyclo-(Arg-Gly-Asp-D-Phe-Lys(^{ε}NH-[CO-(CH₂)₅-NH]₄-Lys-(CO-C₆H₃(CH₂PO₃H₂)₂)₂))
f) cyclo-(Arg-Gly-Asp-D-Phe-Lys(^{ε}NH-[CO-(CH₂)₅-NH]₄-(CO-C₆H₃(CH₂PO₃H₂)₂)₂))
g) cyclo-(Arg-Gly-Asp-D-Phe-Lys(^{ε}NH-[CO-CH₂(-O-CH₂CH₂)₆-NH]₂-Lys-(CO-C₆H₃(CH₂PO₃H₂)₂)₂))
h) cyclo-(Arg-Gly-Asp-D-Phe-Lys (^{ε}NH-[(-O-CH₂CH₂)₆-NH]₂₋Lys-(CO-C₆H₃(CH₂PO₃H₂)₂)₂))
i) cyclo-(Arg-Gly-Asp-D-Phe-Lys(^{ε}NH-[CO-(CH₂)₅-NH]₂-Lys-[Lys-(CO-C₆H₃(CH₂PO₃H₂)₂)₂]₂))
j) cyclo-(Arg-Gly-Asp-D-Phe-Lys(^{ε}NH-[CO-(CH₂)₅-NH]₃-Lys-(CO-C₆H₃(CH₂PO₃H₂)₂)₂)₂))
k) cyclo-(Arg-Gly-Asp-D-Phe-Lys (^{ε}NH-[CO-CH₂(-O-CH₂CH₂)₆-NH]₂-Lys-[Lys-(CO-C₆H₃(CH₂PO₃H₂)₂)₂]₂))

6. Procédé de préparation d'implants enrobés appropriés pour des organes humains et animaux, constitués d'une matrice de support et d'une couche d'une molécule bioactive, médiatrice de l'adhésion cellulaire, enveloppant cette matrice, **caractérisé en ce que** la couche enveloppante est formée à partir d'un composé qui est préparé au moyen d'un procédé selon l'une quelconque des revendications 1 à 5, une liaison ionique ou adsorbante étant présente entre la matrice de support et ce composé.

7. Procédé selon la revendication 6, **caractérisé en ce que** la matrice de support et/ou sa surface est un métal ou un oxyde métallique.

8. Procédé selon la revendication 6, **caractérisé en ce que** la matrice de support et/ou sa surface est une matière de remplacement d'os ou de dent.

9. Procédé selon la revendication 8, **caractérisé en ce que** la matière de remplacement d'os ou de dent est composée de mélanges de phosphate de calcium.
